# EUROPEAN PATENT APPLICATION

(11) **EP 0 646 377 A1**
(43) Date of publication of application: **05.04.1995**
(21) Application number: 93830404.5
(22) Date of filing: 01.10.1993
(51) Int. Cl.: A61K 39/385, A61K 39/00, C07K 16/30, A61K 39/395

(54) **Albumin-conjugated tumour cell-free extracts, antigenic compositions comprising the same, related antibodies, and uses thereof**

(71) Applicant: LUANFARMA S.r.l., I-00142 Rome (IT)
(72) Inventor: Kanduc,Darya, I-00186 Roma (IT)
(74) Representative: de Simone, Domenico

(57) **Abstract**

Compositions comprising as immunising agent tumour cell-free extracts conjugated with albumins are disclosed. Compositions are used for the preparation of pharmaceutical compositions useful as immunostimulants in the therapy of cancer and/or immunodeficiency diseases of humans and animals. Compositions are also used for the preparation of antibodies in experimental animals, to be used for passive immunotherapy of humans and animals.

## Description

This invention relates to compositions comprising tumour cell-free extracts conjugated with albumin proteins, suitable for use as immunogen in the therapy of cancer and/or immunodeficiency diseases of domestic, breeding animals and humans as well. The invention relates also to antibodies raised against said compositions for passive immunotherapy.

Treatment of cancer affected patients by immunologic approaches is known from prior art. A non-specific stimulation of the immune system of such subjects with killed tumour cells has been obtained both in experimental animals and in humans. Killed leukemic cells have been injected into leukemic patients to get a specific immunogenic reaction to leukaemia.

In the attempt to increase the immunogenicity potential, killed tumour cells have been linked to artificial haptens, i.e. trinitrophenol, or have been infected with virus, i.e. Vaccinia virus. The so-modified tumour cells have been successively used to induce an active immune response in animals with experimentally induced tumours (A.K. Abbas, A.L. Lichtman, and J.S. Pober "Cellular and Molecular Immunology" W.B. Saunders Co., Harcourt Brace Jovanovich, Inc. Philadelphia, 1991).

Another approach has been to use soluble tumour antigen extracts prepared from tumours obtained by surgery or by autopsy.

However, when inducing specific immunisation either with intact tumour cells or with cell-free soluble antigenic extracts, soluble antigens released by tumours *in vivo*, block an effective immune response, as a possible undesirable result of injecting exogen antigenic material (R.D. Guttmann et al. "Immunology" The Upjohn Co., Kalamazoo, p. 129, Michigan, 1983).

In any case no definitive results have been achieved in the field.

Albumin proteins, i.e. bovine serum albumin, ovalbumin, rabbit serum albumin, are known from technical literature to exert high immunogenic properties, as carrier of haptens (E. Harlow and D. Lane, "Antibodies" Cold Spring Harbor Laboratory, New York, 1988). However no data have been reported on their ability to induce a specific antigenic anti-tumoral response, when conjugated to tumour cell-free extract components.

It has been surprisingly found that cell-free extracts, either from human tumours of bioptic or autoptic origin or from tumours from experimental animals, show a remarkable immunogenic potential, after conjugation with suitable carriers, i.e. albumin proteins.

The albumin-conjugated tumour cell-free extracts are able to stimulate the natural production of protective anti-tumoral antibodies, once they have been introduced into an host organism.

Therefore compositions comprising the albumin-conjugated tumour cell-free extract of the invention may be advantageously used as immunomodulants in the therapy of immunosuppressed and cancer patients. It has also found that multiple administration of compositions comprising extracts conjugated to albumins of different sources produces different classes of anti-tumour antibodies, thus avoiding the failure of active immunotherapy due to antibodies anti-tumour antibodies in the host.

Specific antibodies raised in experimental animals with the composition of the invention are advantageously suitable for passive immunotherapy of immunodepressed and cancer patients. Multiple administration of antibodies obtained by compositions comprising extracts conjugated to albumins of different sources, thus comprising different classes of anti-tumour antibodies, may be advantageously adopted, avoiding the failure of passive immunotherapy due to antibodies anti-tumour antibodies in the host.

"Albumin protein" is meant albumin of natural or synthetic or recombinant source, originally derived from serum or eggs (ovalbumin) of different species, or fragments thereof having antigenic and/or carrier properties.

"Immunogen" is meant a composition able to induce specific antitumoral antibodies upon introduction into an host.

"Introduction into an host" is meant administering the composition according to the invention, intramuscularly or subcutaneously or intravenously, to a living organism at the amount of 50-500 µg/kg body weight.

"Antigenically different" is meant an albumin protein able to raise antibodies upon introduction into an host, said antibodies non-crossreacting with the endogenous albumin of said host.

The preparation of cell-free tumour extract may be carried out according to methods known to those skilled in the art, by using tumours of human or animal origin and is exemplified in the following examples. The conjugation of albumin to extracts may be carried out according to methods known to those skilled in the art and is exemplified in the following examples.

It is therefore an object of the present invention an albumin-conjugated cell-free tumour extract. Preferably, said albumin-conjugated cell-free tumour extract is able to act as immunogen upon introduction into an host. More preferably said albumin is antigenically different to the endogenous host albumin.

According to a preferred embodiment of the invention, said cell-free tumour extract is obtained from tumours derived from individuals of the same species of said host.

According to a preferred embodiment said albumin comprises more than one albumin protein which is antigenically different to the endogenous host albumin.

According to another preferred embodiment of the invention, said albumin comprises bovine serum albumin (BSA).

According to another preferred embodiment of the invention, said albumin comprises ovalbumin (OA).

According to another preferred embodiment of the invention, said albumin comprises rabbit serum albumin (RSA).

It is another object of the invention the use of the albumin-conjugated cell-free tumour extract for the preparation of a composition suitable as immunogen in the therapy of cancer and/or of immunodeficiency diseases.

It is another object of the invention the use of the albumin-conjugated cell-free tumour extract for the preparation of a composition suitable as immunogen for raising specific antibodies in experimental animals.

It is another object of the invention a composition suitable for use as immunogen upon introduction into an host comprising the albumin-conjugated extract of the invention and a pharmaceutically or veterinarily acceptable carrier.

Preferably said composition further comprises adjuvants suitable for immunisation, more preferably said adjuvant is aluminium hydroxide, most preferably said albumin conjugated tumour cell-free extract and said aluminium hydroxide are in the range of 1:0.4 to 1: 1 wt/wt ratio.

It is another object of the invention an antibody raised against the composition of the invention.

It is another object of the invention a composition comprising the antibody of the invention suitable for use as passive immunotherapeutics in the therapy of cancer and/or of immunodeficiency diseases.

The invention is illustrated in experimental but not limiting examples, wherein:

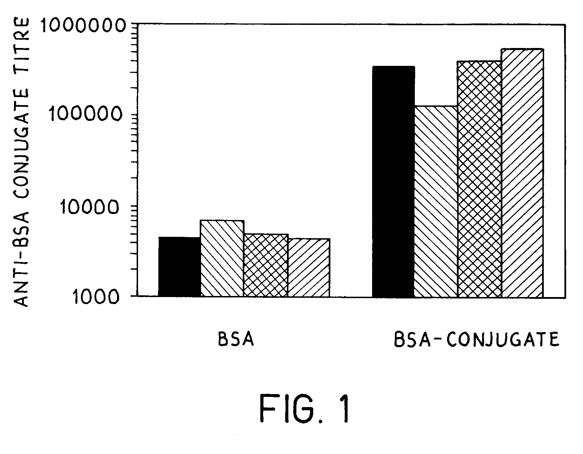

Figure 1 shows IGg titres raised in four groups of rabbits with 5.5 mg of the composition conjugated to BSA; control groups are injected with PBS.

### EXAMPLE 1 Preparation of albumin-conjugated tumour cell-free extracts

All procedures and materials are at 0°-5°.

Tumours (from autopsy or surgery of humans, or induced in animals as described in Solt, D.B., and Farber, E., Nature 263, 701, 1976; Solt, D.B., Medline, A., and Farber, E., Am. J. Pathol. 88, 595, 1977) are cut in 50 mM phosphate buffer saline (PBS), pH 7.4, washed with PBS extensively to remove blood cells, homogenised in a potter by a Waring-Blendor apparatus, sonicated, centrifuged at 10,000 rpm for 10 min, and concentrated by liophylization.

Alternatively different methods are used. Cells may be disrupted either by sonication, or by freeze-thawing, or by treatment with non ionic detergents, as Triton X-100™, or by osmotic shock, or by a combination thereof. Alternatively the lysis buffer may be: (A) 100 mM Tris-HCl, 15 NaCl, pH 7.6; (B) 75 mM NaCl, 25 mM EDTA, 0.1% v/v Triton X-100™.

Cell-free tumour extracts are conjugated to albumin (bovine serum albumin, ovalbumin, or rabbit serum albumin, or fragments thereof having an antigenic and/or a carrier activity) by means of methods known to those skilled in the art. As example, an equal volume of bovine serum albumin solution in 50 mM PBS, pH 7.4, is added to the tumour extract prepared as above, at a 2:1 ratio (2 Absorbance₂₁₅ₙₘ units of bovine serum albumin : 1 Absorbance₂₁₅ₙₘ unit of tumour extract), in 1 ml 50 mM PBS pH 7.4, for each mg albumin. After 10 minutes of stirring, an equal volume of 0.2% w/v glutaraldehyde in PBS is added to the tumour extract/albumin solution, with constant stirring. The reaction mixture is incubated at room temperature for 1h. Glycine is then added to a final concentration of 200 mM, under agitation for 1 hr. The conjugate is dialysed overnight against PBS (8 changes). The solution is lyophilised and stored at a temperature of -80° to 0°.

Alternatively other linking agents know to those skilled in the art may be used, i.e. m-maleimidobenzoyl-N-hydroxysuccinimide or bis-diammonium benzidine.

When required the lyophilised is dissolved in PBS and mixed with suitable amounts of aluminium hydroxide or TiterMax™ (Roberge, F.G., Xu, D. and Chan, C.C., 1992, Current Eye Research, Vol. 11, No. 4, 371-376; Bennett, B., Check, I.J., Olsen, M.R., and Hunter, R.L., 1992 J. Immun. Meth. 153, 31-40) as adjuvant, from 1:0.4 to 1:1 wt/wt.

The same procedures are used when other albumin proteins are employed.

### EXAMPLE 2 Immunostimulating activity of albumin-conjugated tumour extracts

The immune stimulating activity is determined by using standard immunisation procedures. Female albino rabbits are used for the immunisation schedule with albumin-conjugated tumour extracts. When conjugates with rabbit serum albumin are tested, animals from different species (guinea pig or rats) are used. Prior to immunisation, blood specimens to be used as control are withdrawn. The compositions are subcutaneously injected into animals at multiple sites, as a suspension with aluminium hydroxide as described above, at 2.5-7.5 mg in 0.5-1 ml/animal. Control groups receive the adjuvant mixed with BSA in PBS.

Six weeks later, blood specimens are withdrawn. When immunity is achieved, antibodies are isolated from immune sera by immunoaffinity methods. Immune sera and an immunoadsorbent, obtained by coupling of the albumin-conjugated tumour extract to CNBr-activated Sepharose 4B, are mixed at a 1:1 ratio in a minimal volume of PBS and gently shaken at 37°C for 45 min. The mixture is washed in a fritted disk funnel with PBS and retained antibodies are eluted by incubation at 37°C for 30 min with 10% v/v pyridine in PBS, followed by two additional washings with 5 ml of 10% pyridine-PBS. The combined pyridine fractions are dialysed against PBS with several changes of buffer (at least 10). Immunoglobulins (mainly IgGs) are lyophilised and dissolved in a suitable pharmaceutical acceptable solvent (10 mM NaH₂PO₄, pH 7.0, 0.15 mM NaCl) to get a 16% w/v protein solution.

Immune sera are also evaluated for specific antibodies by radio immunoassay using as antigens BSA, adjuvant and tumour extract, separately. The assay is carried out according to known procedures, using 96 well PVC plates, causing the vaccine to adhere, and then adding in successive steps the iodinated immune serum to be analysed, and the iodinated pre-immune control serum. Iodination is carried out by adding the oxidant Chloramine T to a solution of serum and Iodide. Na¹²⁵I (0.5 mCi is added to 10 µg of serum proteins in a total volume of 25 µg of 0.5 M sodium phosphate pH 7.5. Then 25 µl of a fresh solution of 2 mg/ml Chloramine T is added. After incubation for 1 min at r.t., 50 µl of Chloramine T stop buffer are added (2.4 mg sodium metabisulphite, 10 mg/ml saturated tyrosine, 10% glycerol, 0.1% xylene cyanol in PBS). Iodinated serum antibodies are separated from iodotyrosine by gel filtration column using a gel matrix with an exclusion-limit of 20,000-50,000 for globular proteins, medium size beads.

Vaccines are coated to wells by adding 50 µl (approximately 0.5 µg/well) and by incubating for 2 hrs in an humid atmosphere. Wells are washed with PBS. The remaining sites for protein binding on PVC plates are saturated with 3% BSA w/v in PBS with 0.02% sodium azide. After an overnight incubation, plates are washed twice with PBS. The labelled antibody test solution (50 µl) is serially diluted with 3% w/v BSA in PBS, added to each well and incubated for two hours at room temperature in an humid atmosphere. Unbound antibodies are removed by 4 washes with PBS. Then the radioactivity of wells containing immune serum is counted and the radioactivity of control wells with pre-immune serum subtracted.

Antibody titres obtained with groups of 4 rabbits are reported in Figure 1. The results show that vaccines obtained by using tumoral cell-free extracts conjugated to albumin are capable of stimulating the immune system when injected into host animal and induce significant levels of antibodies.

### EXAMPLE 3 Protective effect of tumoral cell extracts conjugated to albumin towards cancer affected subjects

Hepatocarcinoma is induced in albino male rats by using the resistant hepatocyte model developed by Farber et al. (Solt, D.B., and Farber, E., 1976, Nature 263, 701; Solt, D.B., Medline, A., and Farber, E., 1977 Am. J. Pathol. 88, 595). At various stages of the hepatocarcinogenic process, rats are treated with the vaccine obtained by using tumoral cell extract conjugated to bovine serum albumin (a dose of 4.5 mg every 2 months). Then rats are kept under control for 15 months from the beginning of the experiment. The effectiveness of the vaccine is evaluated in terms of survived rats out of a total of 10 animals for each group, as reported in Table 1.

**Table 1**

| Response to the vaccine of rats at various stages of hepatocarcinogenesis | | |
|---|---|---|
| Hepatocarcinogenic stage | Non-treated | Treated |
| Control | 9/10 | 10/10 |
| Nodular liver | 0/10 | 8/10 |
| Neoplastic nodules | 1/10 | 8/10 |
| Hepatocellular carcinoma | 0/10 | 3/10 |

The results reported in Table 1 strongly show that, when the vaccine is administered at early stages of hepatocarcinogenesis process (nodular or neoplastic nodules), it is highly protective towards cancer animals. The effect is less marked when the vaccine treatment is carried out to rats at later stages of hepatocellular carcinoma.

### EXAMPLE 4 Protective effect of rabbit antibodies raised with vaccines obtained using cell-free tumour extracts conjugated to albumin

IgGs (16% w/v solution) isolated from rabbit immune sera obtained with cell-free tumour extracts conjugated to albumin, are administered to rats bearing hepatocellular carcinoma developed according to the resistant hepatocyte model. Each dose (5 mg/kg body weight) is injected through the caudal vein. In all rats are given 10 injections at intervals of 3 days. Then rats are kept under control for 15 months from the beginning of the experiment. The effectiveness of the vaccine is evaluated in terms of survived rats out of a total of 10 animals for each group as reported in Table 2.

**Table 2**

| Response of rats bearing hepatocellular carcinoma to IgGs elicited in rabbits by the vaccine obtained by using extract from hepatocarcinoma conjugated to BSA | | |
|---|---|---|
| Hepatocarcinogenic stage | Non-treated | Treated |
| Control | 9/10 | 10/10 |
| Hepatocellular carcinoma | 0/10 | 7/10 |

The results reported in Table 2 show an increase in the number of survived rats at the last stage of the hepatocarcinogenesis process, following administration of IgG elicited in rabbits by the vaccine obtained by using extract from hepatocarcinoma conjugated to bovine serum albumin.

## Claims

1. Albumin-conjugated cell-free tumour extract.

2. Albumin-conjugated cell-free tumour extract able to act as immunogen upon introduction into an host.

3. Albumin-conjugated cell-free tumour extract according to Claim 2 wherein said albumin is antigenically different to the endogenous host albumin.

4. Albumin-conjugated cell-free tumour extract according to Claim 3 wherein said albumin comprises more than one albumin protein which is antigenically different to the endogenous host albumin.

5. Albumin-conjugated cell-free tumour extract according to any of previous claims wherein said albumin comprises bovine serum albumin (BSA).

6. Albumin-conjugated cell-free tumour extract according to any of claims from 1 to 4 wherein said albumin comprises ovalbumin (OA).

7. Albumin-conjugated cell-free tumour extract according to any of claims from 1 to 4 wherein said albumin comprises rabbit serum albumin (RSA).

8. Albumin-conjugated cell-free tumour extract according to any of previous claims wherein said extract is obtained from tumours derived from individuals of the same species of said host.

9. Use of the albumin-conjugated cell-free tumour extract according to any of previous claims for the preparation of a composition suitable as immunogen in the therapy of cancer and/or of immunodeficiency diseases.

10. Use of the albumin-conjugated cell-free tumour extract according to any of claims from 1 to 8 for the preparation of a composition suitable as immunogen for raising specific antibodies in experimental animals.

11. Composition suitable for use as immunogen upon introduction into an host comprising the albumin-conjugated extract according to any of claims from 1 to 8 and a pharmaceutically or veterinarily acceptable carrier.

12. Composition according to Claim 11 comprising adjuvants suitable for immunisation.

13. Composition according to Claim 12 wherein said adjuvant is aluminium hydroxide.

14. Composition according to Claim 13 wherein said albumin conjugated tumour cell-free extract and said aluminium hydroxide are in the range of 1:0.4 to 1: 1 wt/wt ratio.

15. Antibody raised against the composition according to any of claims from 11 to 14.

16. Composition comprising the antibody according to Claim 15 suitable for use as passive immunotherapeutics in the therapy of cancer and/or of immunodeficiency diseases.
